# EUROPEAN PATENT SPECIFICATION

(11) **EP 4 259 025 B1**
(45) Date of publication and mention of the grant of the patent: **15.04.2026**
(21) Application number: 21848209.9
(22) Date of filing: 08.12.2021
(51) Int. Cl.: A61B 34/20, A61B 17/17, A61B 17/84, A61G 13/00, A61F 2/30, A61F 2/28, A61F 2/32, A61F 2/38, A61B 34/10, A61B 17/72

(54) **FIBER OPTIC CABLE FOR LESS INVASIVE BONE TRACKING**
GLASFASERKABEL FÜR WENIGER INVASIVES KNOCHENTRACKING
CÂBLE À FIBRES OPTIQUES POUR SUIVI OSSEUX MOINS INVASIF

(30) Priority: 09.12.2020 US 202063123187 P
(43) Date of publication of application: 18.10.2023
(73) Proprietor: Smith & Nephew, Inc., Memphis. Tennessee 38116 (US); Smith & Nephew Orthopaedics AG, 6300 Zug (CH); Smith & Nephew Asia Pacific Pte. Limited, Singapore 138565 (SG)
(72) Inventor: FARLEY, Daniel, Cordova, Tennessee 38016 (US); WILSON, Darren J., Hull HU3 2BN (GB); JARAMAZ, Branislav, Pittsburgh, Pennsylvania 15222 (US); BELL, Brett, Pittsburgh, Pennsylvania 15222 (US)
(74) Representative: Appleyard Lees IP LLP
(86) International application number: PCT/US2021/062358
(87) International publication number: WO 2022/125629

(56) References cited:
- EP-A1- 2 116 877
- WO-A1-2011/141829
- WO-A1-2015/038740
- LI XU ET AL: "Dual-layer orthogonal fiber Bragg grating mesh based soft sensor for 3-dimensional shape sensing", OPTICS EXPRESS, vol. 25, no. 20, 28 September 2017 (2017-09-28), pages 24727 - 24734, XP055458601, DOI: 10.1364/OE.25.024727

## Description

### CROSS-REFERENCE TO RELATED APPLICATION

This application claims the benefit of United States Provisional Application Serial No. 63/123,187 filed December 09, 2020, entitled "Fiber Optic Cable and technique for Less Invasive Bone Tracking".

### Field

The present invention relates to a surgical navigation system using fiber optic-based tracking.

### BACKGROUND

In general, computer navigation for orthopedic surgery is used to facilitate alignment for various surgical procedures, such as, fixation of fractures, ligament reconstruction, osteotomy, tumor resection, bone preparation for joint arthroplasty, and verification of implant placement.

One approach to computer navigation uses electromagnetic tracking. Electromagnetic (EM) trackers are small in size (e.g., < 1 millimeter (mm) in diameter) and can be embedded in the bone of a patent. EM navigation systems suffer from a number of disadvantages. Similar to line-of-sight tracking, it can be difficult to maintain an optimal clinical workflow while also satisfying the requirements of the EM system. The EM system only provides accurate measurements within a defined volume with respect to position of the field generator. Further, metallic objects in the sensing field can generate interference and degrade the accuracy of the measurement. In an orthopedic procedure, it is very difficult to avoid distortion and drift as many, if not most, of the instruments used in the procedure are metallic.

Another approach to computer navigation uses fiber optic-based tracking. Fiber optic sensors are non-line of sight and are also immune to RF and EM fields. Thus, fiber optic sensors are an ideal alternative for tracking instruments in confined spaces such as bone tunnels or bone canals.

Tracking instrumentation for computer navigation are rigidly affixed to the patient with a series of cortical pins, which secure the tracking instrumentation to the patient's bony structure. Implantation of the cortical pins requires tissue resection down to the cortex and subsequent drilling and pin driving. While rigid bony fixation of the cortical pins is required for reliable registration and imaging, the pin holes do not serve any purpose other than to secure tracking instrumentation. Furthermore, to prevent occlusion of the tracking instrumentation, the cortical pins are often placed away from the incision site, often in otherwise healthy tissue.

Given the various risks associated with cortical pin placement, many surgeons and insurance providers do not use computer navigation for minimally invasive procedures. This is an unfortunate circumstance as many arthroscopic procedures would benefit from the addition of computer navigation. For example, tunnel placement and graft tensioning for anteromedial portal technique anterior cruciate ligament (ACL) repair could be simplified with enhanced planning and navigation capabilities.

Even where computer navigation is used, such as, total joint replacement surgeries, patients undergoing such procedures would see a benefit from less invasive navigation tracking instrumentation fixation options.

As noted, one type of computer navigation uses fiber optic shape sensing (FOSS). Shape sensing technology uses fiber Bragg grating (FBG) sensors to track position and shape of a surgical instrument, such as a catheter or guide wire, in a spatially continuous manner. A fiber optic Bragg grating (FBG) is a short segment of optical fiber that reflects particular wavelengths of light and transmits others. This is achieved by adding a periodic variation of the refractive index in the fiber core, which generates a wavelength-specific dielectric mirror. A fiber Bragg grating can therefore be used as an inline optical filter to block certain wavelengths, or as a wavelength-specific reflector, which can be correlated with strain data. That is, changes in the fiber's form or position result in changes of the filtered wavelength due to the FBG. For three-dimensional (3D) shape sensing, several fibers (or cores) are disposed around a central fiber (or core), which serves as a reference channel (or reference core). Values from the reference core are compared to the other cores to derive the 3D shape. Then, by combing the FOSS with 3D reconstruction and graph visualization, the 3D shape of the flexible instrument can be visualized.

As an alternative to fiber-optic Bragg gratings, the inherent backscatter in conventional optical fibers can be exploited. One such approach is to use Rayleigh scatter in standard single-mode communications fiber. Rayleigh scatter occurs as a result of random fluctuations of the index of refraction in the fiber core. These random fluctuations can be modeled as a Bragg grating with a random variation of amplitude and phase along the grating length. By using this effect in three or more cores running within a single length of multicore fiber, the 3D shape and dynamics of the surface of interest can be followed.

For computer navigation during orthopedic procedures, cables housing these fiber cores must be attached at the structure to be tracked, such as, a patient's bone. However, where several instruments and/or locations of the patient are being tracked, the cables may obstruct the operating room workspace. Thus, the operating room workspace may be impeded by a number of wires and various instruments that may be caught on other structures in the workspace.

In addition, the manner in which the fiber is integrated into a device or attached to the bone can affect its performance as a tracking modality in surgical navigation in terms of displaying the relative position and orientation of these anatomical structures. For example, conventional FOSS use cables that are generally prone to twisting and kinking. Large amounts of twisting or kinking can lead to inaccuracies in measurements over time. Additionally, the fiber cores rely on tension and compression applied to the sensors, as such wear on the fiber (e.g., from twisting and kinking, etc.) may lead to inaccuracies over time and quicker failure of the fiber cores. Additionally, still, a large amount of twisting or kinking may place a strain on the patient's bony structure, especially where rigidly affixed to the bone.

Despite these drawbacks, it is important to rigidly affix the fiber cores to the patient's bone in order to obtain accurate measurements as the determination of the position of the bone is based on locating the distal tip of the fiber core. WO 2015/038740 A1 describes a method of receiving shape data from an optical fiber section in a shape sensor.

### BRIEF SUMMARY

Thus, it would be beneficial to provide navigation tracking instrumentation fixation options that are less invasive than current techniques. Further, it would be beneficial to provide cables and fiber cores that are less prone to twisting and kinking reducing potential sources of error. It is with this in mind that the present disclosure is presented.

For example, an advantage of optical fiber-based tracking, in accordance with the present disclosure, over line-of-sight optical tracking, is the low size and weight of the sensors. This means that the sensor can be attached to the patient anatomy in ways more favorable to the patient. For example, reducing the size, depth, and number of screw holes can improve the patient's recovery, reduce complications such as fractures and infection, and may improve adoption among clinicians.

The present disclosure provides a surgical navigation system based on an optical fiber carrying lumen that is configured to improve shape sensing performance by dampening vibrations from an external environment, providing a smooth, continuous and pinch-free lumen, and permitting the fiber to slide freely within the lumen. Shape sensing performance is also improved by decoupling torque of the device from the twisting of the fiber. The surgical navigation system utilizes multimodal tracking along with low profile/small diameter bone pins to fix FBG sensors to a patient. Limited cross-sectional area is available inside many interventional devices such as bone pins. A significant challenge is presented to create an optimal lumen for a fiber given the limited space available in the cross-sectional footprint of a bone pin. Optical fiber dimensions are on the order of hundreds of microns on an outer diameter. In many cases, interventional devices include a guide-wire channel or a bone pin within a small cross-sectional area, e.g., about 2.1 mm in the case of a 6 French catheter. The present embodiment overcome this space limitation by configuring existing features of medical devices to create a lumen for the optical shape sensing fiber.

With some embodiments, FBGs may also use Fresnel reflection at each of the interfaces where the refractive index is changing. For some wavelengths, the reflected light of the various periods is in phase so that constructive interference exists for reflection and, consequently, destructive interference for transmission.

Protection and isolation from the external environment in theatre are a prerequisite for FOSS in surgical navigation, which employs a calculation of strain along a multicore optical fiber to reconstruct the shape along the fiber. As such, the shape stability and reconstruction accuracy are susceptible to changes in tension, twist, vibration, and/or pinching. Integrating this technology into interventional devices used in a dynamic environment, such as that of orthopedic navigation, can cause significant degradation of FOSS (e.g., due to vibration action of the handheld burr during resection of the bone tissue, among other reasons).

With regard to decoupling of twist, the accuracy of the optical shape sensing position degrades with increased twist along the length of the sensor. Since torquing of medical instruments is common in many procedures, there is considerable value in designing devices to decouple or reduce the torquing of the device from twisting of the sensors. With careful selection of the lumen position and properties, it is possible to decouple the instrument torquing from the twisting of the fiber.

With some embodiments, a multi-core fiber optic cable having both an infrared (IR) tracking sensor disposed at a known location in the multi-core fiber optic cable and one or more FBGs, is provided. The FBGs can be used to locate the tip of the cable relative to the IR marker and where the tip of the cable is embedded in a bone, the location of the bone can be determined.

The surgical navigation system and the multi-core fiber optic cable described herein could be used for navigated orthopedic procedures, such as, total knee replacement (TKA), total hip replacement (THA), patellofemoral knee replacement (PFA), unicompartmental knee arthroplasty (UKA), or the like.

In at least one example embodiment, a patient tracking system is disclosed. The patient tracking system may be less invasive than conventional patent tracking systems, and in some examples is referred to as a non-invasive patient tracking system. The non-invasive patient tracking system comprises one or more optical fiber cables comprising a first end, a middle, and a second end. The middle being separated from the second end by a tracked portion. The non-invasive patient tracking system further comprising at least one optical interrogator optically coupled to the first end. The non-invasive patient tracking system additionally comprising a non-invasive tracking array mechanically coupled to the middle, wherein the non-invasive tracking array is trackable via a global tracking device and a surgical tool mechanically coupled to the second end. The non-invasive patient tracking system additionally comprises a processor and a non-transitory, processor-readable storage medium that stores instructions executable by the processor to obtain, from the global tracking device, locational data associated with the non-invasive tracking array and to obtain, from the optical interrogator, indications of a fiber optic shape associated with the tracked portion of each of the one or more optical fiber cables. The instructions, when executed, further cause the processor to determine, based on the locational data and the fiber optic shape, a location of the surgical tool.

In one embodiment, the one or more optical fiber sensors are connected to bones or other anatomical features using an attachment device. The attachment device may include a plurality of different configurations including bone screws, pins, cements, adhesives, clamps, etc. The one or more optical sensors may also be connected to a surgical instrument, which may include a pointer, a catheter, a guidewire, a probe, an endoscope, a robot arm, a drill, a cutting rig or other medical component, etc.

In the context of registration, several coordinate systems are proposed. These include an optical shape sensing coordinate system (OSSCS) that may be attached to a fixture within the operating room. In a TKR procedure, a femoral coordinate system (FCS) is local to the femur, and a tibial coordinate system (TCS) is local to the tibia. A pointer (or any other instrument) coordinate system (PCS) is local to an instrument.

The first coordinate systems (OSSCS) serves as a reference coordinate system for both registration and navigation. FCS, TCS and PCS are moving relative to OSSCS. Transformations between FCS, TCS, PCS and OSSCS are known through fixed transformations between the fibers at the launch point and changing transformations between fiber tip and launch point obtained through the shape sensing of the fiber.

In one embodiment, the non-invasive tracking array comprises fiber Bragg gratings (FBG).

In one embodiment, the non-invasive tracking array comprises fiber Bragg gratings arranged in a grid to form a mesh, wherein the mesh can be overlaid onto a surface of a patient's bone.

There are multiple ways in which the optical fiber sensor can be attached to the bone, which trade off invasiveness with precision of tracking. For example, a bone screw is the most invasive approach, but can provide the most rigid fixation, while a skin adhesive is the least invasive, but offers less precise tracking of the bone position. The preferred fixation approach may depend on the accuracy requirements of the application. For example, a bone cement can be used to secure the optical fiber in place during the procedure. After the procedure, the cement is either left in place or removed from the bone. Alternatively, a unicortical pin takes advantage of the properties of the optical fiber since the optical fiber is a very light component that does not need deep fixation into the bone. A unicortical pin shaft may include a cannulation that allows insertion of the optical fiber.

In one embodiment, the surgical tool is an IM nail.

In one embodiment, the surgical tool is a cannulated pin.

In at least one embodiment, a fiber optic tracking system is disclosed. The fiber optic tracking system comprises a partially hollow bone pin comprising a tip end, a driving end, and a first coupling device positioned between the tip end and the driving end, the partially hollow bone pin being configured to be anchored into a patient's bone. The fiber optic tracking system further comprises a fiber sheath comprising one or more optical fiber cables, and a second coupling device complementary to the first coupling device. The fiber sheath having a shape configured to securely fit within the partially hollow bone pin. The one or more optical fiber cables comprising a first end and a second end, wherein the second end is a known distance from the tip end. The fiber optic tracking system additionally comprising at least one optical interrogator optically coupled to the first end, a processor, and a non-transitory, processor-readable storage medium that stores instructions executable by the processor. The instructions, when executed by the processor, cause the processor to obtain, from the optical interrogator, indications of a fiber optic shape associated with each of the one or more optical fiber cables and determine, based on the fiber optic shape and the known distance, a location of the tip end.

Embodiments of the present disclosure provide numerous advantages. For example, when compared to conventional IR surgical navigation systems, the use of small diameter FBG sensors is far less invasive than the use of half-pins. This reduces the risk of iatrogenic injury resulting from infection, vascular damage, nerve injury, and periprosthetic fracture and postoperative deep venous thrombosis. Furthermore, the embodiments of the present disclosure have the potential to improve operating room ergonomics reducing the opportunity for interferences between the surgical team and the navigation system. In conventional navigated knee replacement procedures, the femur and tibia are tracked independently, where each is outfitted with a reflective reference array. Therefore, both arrays must remain visible during the entire procedure. However, the embodiments of the present disclosure require only a single reflective reference array, which can be placed away from the patient in a high visibility area, so the surgeon does not have to be concerned with marker occlusion.

Furthermore, the present disclosure provides advantages in that the cables disclosed herein will generally lay flat while conventional cables (e.g., rounded, or the like) may be prone to twisting and kinking. The mitigation of cable twisting and kinking increases the accuracy of the tracking systems disclosed herein as the system does not need to predict and/or account for random twisting and kinking that may occur during registration and tracking within the sterile field and be a potential source of error. Thus, the resultant shape data is more accurate due to the redundancy in unforeseen twisting and kinking that may occur within the workflow. Furthermore, mitigating cable twisting and kinking reduces clutter in the operating room and also reduces the likelihood that the cables will catch or get caught on other instruments or structures in the operating room. This is a significant advantage where multiple fibers are being used to track multiple objects. Furthermore, premature wear and/or fracture of the fiber optic cables is prevented by reducing the amount or number of twists and kinks exerted on the cables. Furthermore, reducing the amount or number of twists and kinks can also reduce torsion or strain on the bone pin, and thus the patient's bone is also subjected to less twisting and kinking.

Further features and advantages of at least some of the embodiments of the present disclosure, as well as the structure and operation of various embodiments of the present disclosure, are described in detail below with reference to the accompanying drawings.

### BRIEF DESCRIPTION OF THE SEVERAL VIEWS OF THE DRAWINGS

To easily identify the discussion of any particular element or act, the most significant digit or digits in a reference number refer to the figure number in which that element is first introduced.

It is noted, the drawings are not necessarily to scale. The drawings are merely representations, not intended to portray specific parameters of the disclosure. The drawings are intended to depict example embodiments of the disclosure, and therefore are not considered as limiting in scope. In the drawings, like numbering represents like elements.

Furthermore, certain elements in some of the figures may be omitted for illustrative clarity. The cross-sectional views may be in the form of "slices", or "near-sighted" cross-sectional views, omitting certain background lines otherwise visible in a "true" cross-sectional view, for illustrative clarity. Furthermore, for clarity, some reference numbers may be omitted in certain drawings.
FIG. 1 illustrates a multi-core fiber optic cable 100, in accordance with embodiment(s) of the disclosure.
FIG. 2 illustrates a multi-core fiber optic cable 200, in accordance with embodiment(s) of the disclosure.
FIG. 3 illustrates a portion 300 of a multi-core fiber optical cable, in accordance with embodiment(s) of the disclosure.
FIG. 4A illustrates a portion of a multi-core fiber optic cable 400, in accordance with embodiment(s) of the disclosure.
FIG. 4B illustrates another view of a portion of multi-core fiber optic cable 400, in accordance with embodiment(s) of the disclosure.
FIG. 4C illustrates yet another view of a portion of multi-core fiber optic cable 400, in accordance with embodiment(s) of the disclosure.
FIG. 5 illustrates a cable pin assembly 500, in accordance with embodiment(s) of the disclosure.
FIG. 6 illustrates a cortical pin 600 for cable pin assembly 500, in accordance with embodiment(s) of the disclosure.
FIG. 7A illustrates a view of cable pin assembly 500, in accordance with embodiment(s) of the disclosure.
FIG. 7B illustrates another view of cable pin assembly 500, in accordance with embodiment(s) of the disclosure.
FIG. 7C illustrates another view of cable pin assembly 500, in accordance with embodiment(s) of the disclosure.
FIG. 8 illustrates a surgical navigation system 800, in accordance with embodiment(s) of the disclosure.
FIG. 9A illustrates a system 900 for determining placement of an IM nail, in accordance with embodiment(s) of the disclosure.
FIG. 9B illustrates elements of system 900, in accordance with embodiment(s) of the disclosure.
FIG. 9C illustrates features of system 900, in accordance with embodiment(s) of the disclosure.
FIG. 10 illustrates modeling an anatomy of a patient's bone with an FBG mesh, in accordance with embodiment(s) of the disclosure.
FIG. 11A illustrates an FBG mesh 1100, in accordance with embodiment(s) of the disclosure.
FIG. 11B illustrates another view of FBG mesh 1100, in accordance with embodiment(s) of the disclosure.
FIG. 11C illustrates yet another view of FBG mesh 1100, in accordance with embodiment(s) of the disclosure.
FIG. 12A illustrates a shape to be modeled, in accordance with embodiment(s) of the disclosure.
FIG. 12B illustrates a shape model associated with the shape of FIG. 12A, in accordance with embodiment(s) of the disclosure.
FIG. 12C illustrates a shape to be modeled, in accordance with embodiment(s) of the disclosure
FIG. 12D illustrates a shape model associated with the shape of FIG. 12C, in accordance with embodiment(s) of the disclosure.
FIG. 12E illustrates a shape to be modeled, in accordance with embodiment(s) of the disclosure
FIG. 12F illustrates a shape model associated with the shape of FIG. 12E, in accordance with embodiment(s) of the disclosure.
FIG. 13 illustrates a routine 1300 in accordance with one embodiment.
FIG. 14 illustrates a diagrammatic representation of a machine 1400 in the form of a computer system within which a set of instructions may be executed for causing the machine to perform any one or more of the methodologies discussed herein, according to embodiment(s) of the disclosure.
FIG. 15 illustrates a computer-readable storage medium 1500, in accordance with embodiment(s) of the disclosure.

### DETAILED DESCRIPTION

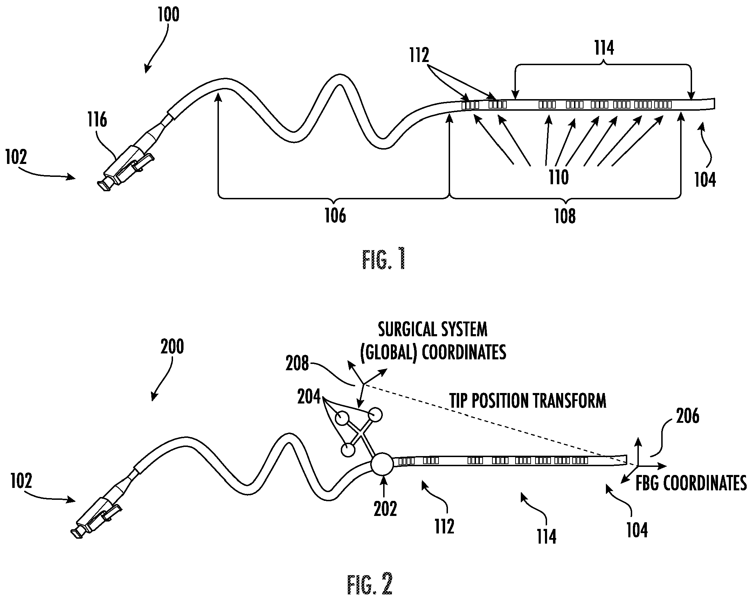

FIG. 1 illustrates a multi-core fiber optic cable 100, in accordance with non-limiting example(s) of the present disclosure. In general, multi-core fiber optic cable 100 is arranged to provide indications of six degrees-of-freedom (6DoF) pose (e.g., orientation and position) estimation of a patient's bony structure for use either during surgical navigation or robotic-assisted surgery systems.

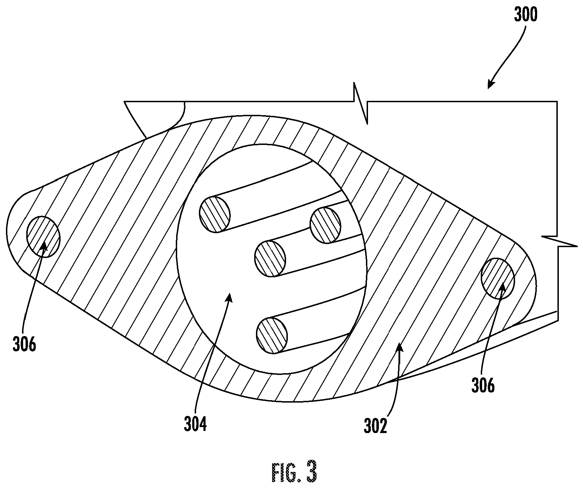

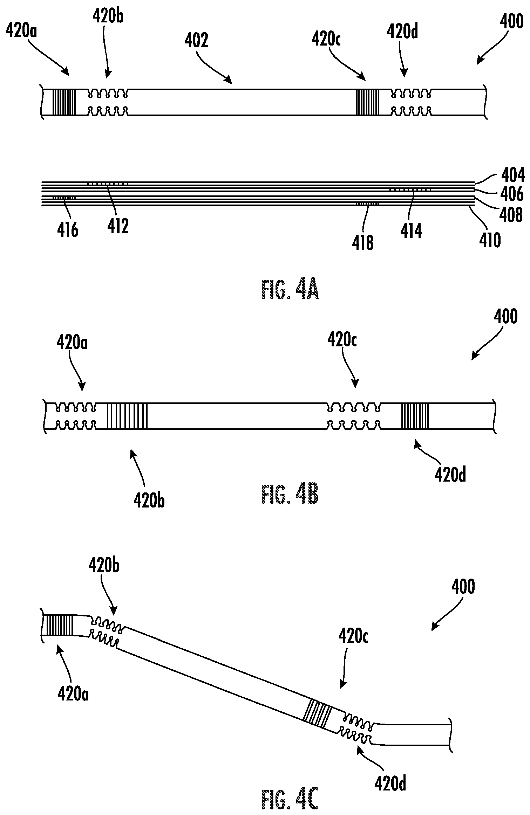

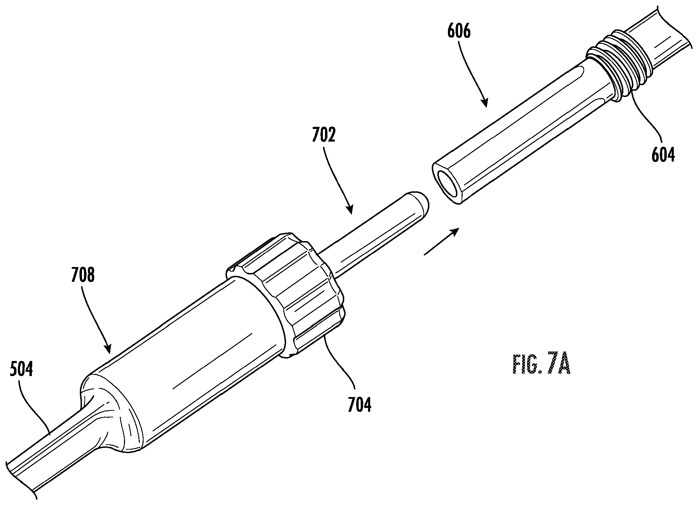

As depicted, multi-core fiber optic cable 100 includes a first end 102 and a second end 104 separated by a lead portion 106 and a sensor portion 108. Multi-core fiber optic cable 100 includes a fiber optic bundle or a number of optical fibers (see FIG. 3, FIG. 4A, FIG. 4B, and FIG. 4C). The sensor portion 108 includes a number of fiber Bragg grating (FBG) sensors, such as, FBG sensors 110. The FBG sensors 110 are grouped into reference sensors 112 and tracking sensors 114. The sensor portion 108, including reference sensors 112 and tracking sensors 114, is proximate to the second end 104. In some examples, as will be described in greater detail below, tracking sensors 114 can be at the second end 104, or even exposed at the second end 104. For example, the distal tip of multi-core fiber optic cable 100 (e.g., the distal tip of second end 104) can be exposed from a shroud (e.g., FIG. 7A) such that, when the cable is utilized intraoperatively, the tip of multi-core fiber optic cable 100 may be embedded into a patient's bone through a cannulated pin or wire for local tracking of the pose of the bone.

With some design considerations, this bone pin can also be made to dampen vibration and can be fabricated to minimize friction on its inner surface. Such considerations may include the addition of coatings on the inner diameter of the lumen of the bone pin. These coatings may include Teflon^{™}, PTFE, MDX, Pebax^{™}, or other substances to reduce friction. The bone pin may include a dampening material or coating configured to provide vibration-dampening features. The bone pin may be vibrationally damped by being coiled or made from materials with dampening properties, etc.

Another benefit of the design is that the optical fiber lies along a center of the device (neutral axis), which means that there will be minimal path length changes along the fiber during bending of the device (thereby reducing the amount of motion, friction, and strain that the fiber experiences during bending). In addition, since the fiber lies within the torquing element of the device and along a central axis, it is rotationally free to slide in the lumen of the bone pin and will be isolated from external torquing, unlike the case where the fiber is off-axis where torquing of the device will necessarily cause the fiber to twist as it is offset from the axis of rotation.

Although many embodiments described herein describe cortical pin fixation for the multi-core fiber optic cable 100, alternative fixation techniques could be utilized, such as, for example, an anti-rotation device and a bone pin, multiple pins and a screw base, or the like.

Lead portion 106 is disposed between the sensor portion 108 and the first end 102. Multi-core fiber optic cable 100 also includes a coupler 116, disposed on first end 102 proximate to the lead portion 106 and distal to the sensor portion 108. The length of lead portion 106 may be selected such that coupler 116 can be physically coupled to components of a surgical navigation system and the sensor portion 108 can be fixed to a patient's bony structure.

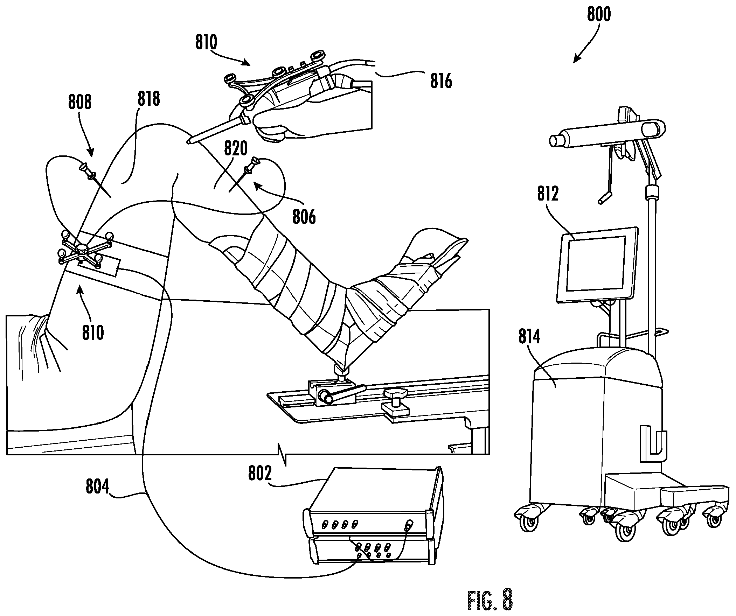

Coupler 116 is arranged to couple multi-core fiber optic cable 100 to a surgical navigation system (e.g., FIG. 8, or the like) to provide optical communication between the optical fibers of multi-core fiber optic cable 100 and a signal generator. This is described in greater detail below. However, in general, coupler 116 provides that optical signals are communicated between a signal generator and FBG sensors 110 via optical fibers of multi-core fiber optic cable 100. A surgical navigation system can determine, based on these signals, the position and orientation of a patient's bony structure in three-dimensional space.

For example, feedback from the tracking sensors 114 relative to feedback of the reference sensors 112 can be used to determine the position and orientation of each of the tracking sensors 114. More specifically, individual locations of each FBG sensor 110 in multi-core fiber optic cable 100 can be determined. Where these portions of multi-core fiber optic cable 100 are fixed (e.g., via cortical pins, or the like) to a patient's bony structure, the position and orientation of the patient's bony structure can be determined. Said differently, the sensor portion 108 can be fixed (e.g., embedded via cortical pins, or the like) to a patient's bone. As such, the orientation and position of the bone can be determined and a representation (e.g., three-dimensional (3D), or the like) of the bone can be generated without traditional techniques, such as, computed tomography (CT) scans, or the like.

The main configurations of FBGs-based shape sensors can be grouped into multiple single-core fibers, and multicore fibers (MCFs), having several cores integrated into a single fiber. The most widely used configurations for shape sensing would be a triangular with three outer cores, square with four outer cores and hexagonal with one central core and six outer core. The more cores that are exploited, the higher is the accuracy of the shape sensor at the equal value of core spacing (core-to-core distance). The triangular configuration is the preferred embodiment given that it can be interrogated using only three channels of the interrogation unit and ensures a simpler assembly than the other configurations. Furthermore, when employing a central core, such configuration allows twist angles detection in addition to temperature compensation, axial strain removal, and curvature sensing.

The number of FBGs written in each fiber influences the spatial resolution of the sensor. The larger the FBG density, spaced by as little as 1 mm, the greater the spatial resolution (<1mm), which allows the reconstruction of more complex shapes such as bone tunnels in ACL procedures.

Multi-core fiber optic cable 100 could be implemented in tandem with conventional robotic assisted surgical systems or conventional surgical navigation systems, such as, those, which utilize infrared (IR) tracking. This allows the shape sensing technology to be integrated and overlaid with existing imaging modalities. Furthermore, multi-core fiber optic cable 100 could include a single optical fiber core with multiple attachment points (e.g., an attachment point for each FBG sensor of tracking sensors 114, or the like). In other examples, FBG sensors 110 can include multiple optical fibers (e.g., one (1) optical fiber per bone, or the like).

Thus, multi-core fiber optic cable 100 provides an advantage over conventional systems in that fixation of multi-core fiber optic cable 100 to the patient's bony structure is less invasive than conventional options. In particular, FBG sensors (e.g., FBG sensors 110, or the like) are small in diameter (<1 mm) and therefore can be embedded in a cannulated, small diameter cortical pin. This is far less invasive than the multiple half-pins that are used to secure reflective optical marker arrays to a patient in conventional systems.

FIG. 2 illustrates a multi-core fiber optic cable 200, in accordance with non-limiting example(s) of the present disclosure. Multi-core fiber optic cable 200 can be like multi-core fiber optic cable 100 but further include a surgical system reference sensor 202. In some embodiments, surgical system reference sensor 202 can be an IR sensor or IR marker. In another embodiment, surgical system reference sensor 202 can be an electromagnetic tracker. For example, an electromagnetic sensor could be placed in-line with the tracking sensors 114 (e.g., FBG sensors 110, or the like) and could be used to locate the bony segments relative to a surgical navigation or robotic assisted surgical system.

For example, during use, the position of surgical system reference sensor 202 can be determined, for example, by a surgical navigation system (e.g., FIG. 8, or the like). Additionally, the position and orientation of the second end 104, or more specifically, the position an orientation of the FBG sensors 110 of tracking sensors 114 can be determined relative to the position of surgical system reference sensor 202. Accordingly, multi-core fiber optic cable 200 can be used with a surgical navigation system or a robotic surgery system.

FIG. 3 illustrates a cut-away view of a portion 300 of a multi-core fiber optic cable, in accordance with non-limiting example(s) of the present disclosure. Portion 300 can be a portion of multi-core fiber optic cable 100, multi-core fiber optic cable 200, or another multi-core fiber optic cable according to embodiments of the present disclosure. For illustrative purposes only, and not by way of limitation, portion 300 is described with reference to multi-core fiber optic cable 100.

As depicted, portion 300 of multi-core fiber optic cable 100 includes a cable shroud 302 (or sheath) encasing optical fibers 304. Each of optical fibers 304 can include any number of FBGs 110. In this figure, optical fibers 304 are depicted including a respective FBG sensors 110, mainly for purposes of clarity. However, it is noted that a single fiber optic strand 304 can include multiple FBG sensors 110. As a specific example, a multi-core fiber optic cable 100 could have four (4) fiber cores (e.g., optical fibers 304) each having multiple FBG sensors 110 (e.g., between 2 and 20, 2, 4, 6, 8, 10, 12, or the like). In other embodiments, more or less fiber cores and respective FBGs 110 than stated above can be disposed in a multi-core fiber optic cable. Further, one fiber optic strand 304 can include a different number of FBGs 110 than another fiber optic strand 304. Additionally, although not depicted herein, the multi-core fiber optic cable 100 terminates at an interrogator that reads changes in the light wavelengths (e.g., resulting from the FBG sensors 110, or the like).

In one embodiment, cable shroud 302 can have a flattened shape. That is, cable shroud 302 can have a cross-section that is longer in one direction than another, where the directions are substantially orthogonal to each other. As a specific example, cable shroud 302 can be elongated in a radial direction (i.e., orthogonal to the axis of the FBG sensors 110) and have a width greater than its height. In one embodiment, cable shroud 302 may have a width to height ratio of greater than or equal to 2:1, greater than or equal to 3:1, or the like. An advantage to the cable shroud 302 having such a flattened shape is that twisting of the cable along its length is reduced. Furthermore, as a result of the elongated cross-section of the cable shroud 302, a greater force is required to rotate a portion of the multi-core fiber optic cable 100 relative to an adjacent portion of the multi-core fiber optic cable 100 than would typically be required for a similar multi-core fiber optic cable with a round shroud. Thus, multi-core fiber optic cable 100 having cable shroud 302 is biased to align the orientation of the cable shroud 302 along the length of the multi-core fiber optic cable 100.

Cable shroud 302 is formed of any of a variety of materials. the materials with which cable shroud 302 are formed can be selected to, for example, allow some bending and rotating of the multi-core fiber optic cable 100 that is inherent to the use of the multi-core fiber optic cable 100 for tracking of bone pose during surgical navigation.

Multi-core fiber optic cable 100 can further include cable shroud stiffeners 306 disposed in cable shroud 302 along a length of multi-core fiber optic cable 100 to further stiffen or strengthen cable shroud 302. It is noted that cable shroud stiffeners 306 are optional and multi-core fiber optic cable 100 could be provided with cable shroud 302 not having cable shroud stiffeners 306. Additionally, in some embodiments, cable shroud stiffeners 306 might be provided along only selected portions (e.g., lead portion 106, sensor portion 108, or the like) of multi-core fiber optic cable 100

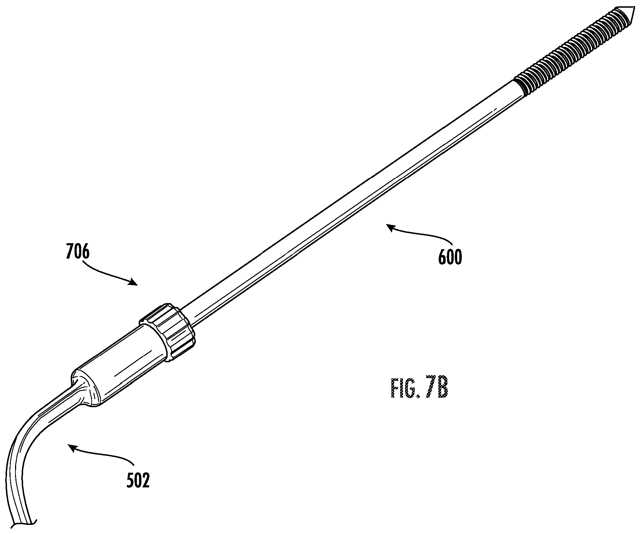

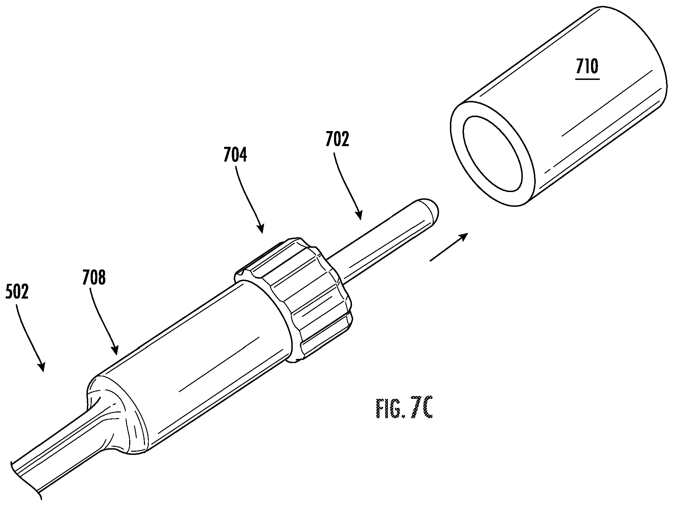

The distal end of the shroud may also include a mating feature for attachment to a cannulated bone pin or other bone fixation component in order to insert and fix the multicore fiber (e.g., FIG. 7A, FIG. 7B, and FIG. 7C).

FIG. 4A, FIG. 4B, and FIG. 4C illustrate views of a portion of a multi-core fiber optic cable 400. The multi-core fiber optic cable depicted in these figures, that is, multi-core fiber optic cable 400, can be like multi-core fiber optic cable 100 and/or multi-core fiber optic cable 200, and in particular, can include the elements and features of the multi-core fiber optic cables described herein. Turning particularly to FIG. 4A, a portion of multi-core fiber optic cable 400 is depicted showing a top view and an internal cut-away view of multi-core fiber optic cable 400. Multi-core fiber optic cable 400 includes a cable shroud 402 and a number of optical fibers or fiber cores, disposed within the cable shroud 402. In particular, multi-core fiber optic cable 400 include four (4) fibers cores, specifically, fiber core 404, fiber core 406, fiber core 408, and fiber core 410.

Multi-core fiber optic cable 400 additionally includes FBGs. As depicted, multi-core fiber optic cable 400 includes an FBG 412 formed in fiber core 404, FBG 414 formed in fiber core 406, FBG 416 formed in fiber core 408, and FBG 418 formed in fiber core 410. As detailed above, FBGs (e.g., FBG 412, FBG 414, FBG 416, and FBG 418) can be utilized to locate the tip of the fiber core (e.g., which can be embedded in a patient's bone via a cortical pin, or the like).

Multi-core fiber optic cable 400 further includes channels formed in cable shroud 402 to provide regions of flexibility in the cable shroud 402. In particular, multi-core fiber optic cable 400 can include channels 420a, channels 420b, channels 420c and channels 420d formed in cable shroud 402. In some embodiments, channels can be formed in regions near FBGs. For example, channels 420a are formed in cable shroud 402 adjacent to FBG 416, channels 420b are formed in cable shroud 402 adjacent to FBG 412, channels 420c are formed in cable shroud 402 adjacent to FBG 418, and channels 420d are formed in cable shroud 402 adjacent to FBG 414.

Turning now to FIG. 4B, a side view of multi-core fiber optic cable 400 is depicted. As can be seen, channels 420a, channels 420b, channels 420c, and channels 420d are formed provide selective flexibility in cable shroud 402. In particular, channels 420a and channels 420c are arranged to provide flexibility in a first direction while channels 420b and channels 420d are arranged to provide flexibility in a second direction different (e.g., orthogonal, or the like) from the first direction.

Turning more particularly to FIG. 4C, a top view of multi-core fiber optic cable 400 bent or manipulated taking advantage of the flexible regions provided by the channels. In particular, multi-core fiber optic cable 400 is depicted bent at regions of cable shroud 402 where channels are formed. For example, cable shroud 402 is bent, primarily at channels 420b and channels 420d.

Although cable shroud 402 is depicted with channels arranged to provide selective flexibility, other means of providing selective flexibility may be implemented. For example, a multi-core fiber optic cable according to the present disclosure could be provided with a shroud formed of interlocking components (e.g., loc-line, gooseneck, or the like) arranged to provide flexibility of the shroud but prevent free-twisting of the shroud.

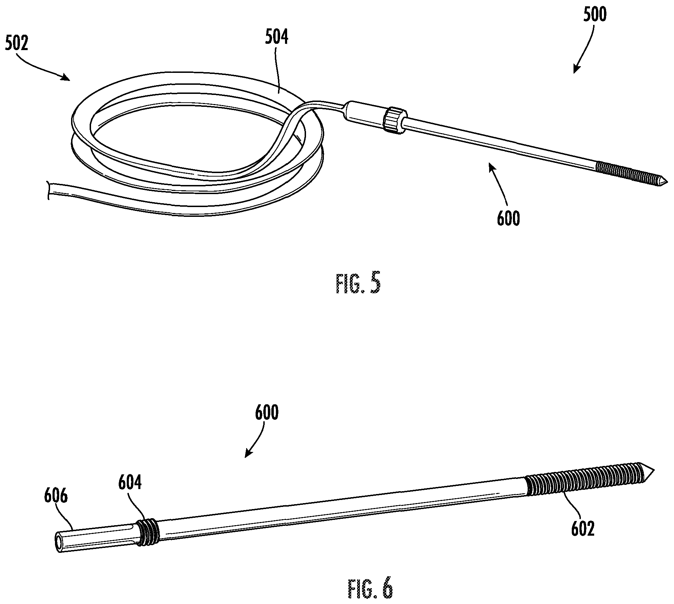

FIG. 5, FIG. 6, FIG. 7A, FIG. 7B, and FIG. 7C illustrates a cable pin assembly 500, in accordance with non-limiting example(s) of the present disclosure. As depicted, cable pin assembly 500 includes a multi-core fiber optic cable 502 and a cortical pin 600. In some embodiments, multi-core fiber optic cable 502 can be like the multi-core fiber optic cable 100, multi-core fiber optic cable 200, and/or multi-core fiber optic cable 400. In particular, multi-core fiber optic cable 502 includes a cable shroud 504 housing fiber cores and FBGs for use in locating a pose or position of a patient's bony structure as described above. That is, cortical pin 600 can be fixed to a patient's bone and multi-core fiber optic cable 502 can be coupled to a surgical navigation system.

Turning to FIG. 6, cortical pin 600 can include external threads 602, cable coupling threads 604, and a canal 606. In some embodiments, cortical pin 600 can be a half-pin. In general, cortical pin 600 can have any suitable diameter. However, with some examples, cortical pin 600 can have a diameter of 4 millimeters (mm) or a diameter between 2 and 6 mm.

External threads 602 can be used to fix (e.g., embed) cortical pin 600 to (or in) a patient's bone to be located during a surgical navigation procedure. Cable coupling threads 604 can be used to couple cortical pin 600 to multi-core fiber optic cable 502 (e.g., as depicted in FIG. 7A and FIG. 7B) while canal 606 can house an exposed fiber tip 702 of multi-core fiber optic cable 502 such that the end of the fiber core passes partially through canal 606 of the cannulated cortical pin 600. In particular, exposed fiber tip 702 extends out of cable shroud 504.

Turning to FIG. 7A, FIG. 7B an exploded view of the mating between multi-core fiber optic cable 502 and 600 is depicted. When multi-core fiber optic cable 502 and cortical pin 600 are coupled, exposed fiber tip 702 passes partially into canal 606 of cortical pin 600.

With some examples, cortical pin 600 can be inserted or fixed to the patient's bone prior to multi-core fiber optic cable 502 being coupled to cortical pin 600. Furthermore, in some embodiments, cortical pin 600 is inserted into the patient's bone a known depth. Then given the geometry of the cortical pin 600 and, and the known depths with which the cortical pin 600 is inserted into the patient's bone as well as the depth with which exposed fiber tip 702 passes through canal 606, the exact location of the tip of the cortical pin 600 as well as its orientation can be determined. It is noted, that with the cable pin assembly 500 described herein, the fiber tip does not actually touch the patient's bone.

The distal end of the cable shroud 504 of multi-core fiber optic cable 502 may include an element arranged to mate with cortical pin 600. For example, multi-core fiber optic cable 502 is depicted including cortical pin coupler 704 arranged to couple to cable coupling threads 604 of cortical pin 600. For example, the cable shroud 504 of multi-core fiber optic cable 502 may include an internally threaded luer connector configured to mate with cable coupling threads 604 of cortical pin 600. Thus, cortical pin coupler 704 can be twisted (e.g., 706) to lock multi-core fiber optic cable 502 to cortical pin 600.

With some examples, the cable shroud 504 of multi-core fiber optic cable 502 can be free spinning with respect to the cortical pin coupler 704 such that the shroud of multi-core fiber optic cable 502 does not twist once locked to cortical pin 600. Additionally, in some embodiments, cable shroud 504 includes a widened distal region 708 to accommodate the proximal end of cortical pin 600 to a sufficient length depth for cable coupling threads 604 to bottom out in the threads of cortical pin coupler 704.

Turning specifically to FIG. 7C, cable pin assembly 500 can include a dust cap 710 arranged to mate with the end of multi-core fiber optic cable 502 comprising the exposed fiber tip 702 and to cover exposed fiber tip 702 when multi-core fiber optic cable 502 is not in use. For example, although not depicted, dust cap can include threads to mate with corresponding threads on cortical pin coupler 704. As another example, dust cap 710 can be a plastic sleeve arranged to slide over cortical pin coupler 704. Dust cap 710 can protect the ferrule of exposed fiber tip 702 from contact with contamination that can scratch, chip, crack or physically damage the polished fiber prior to assembly with cortical pin 600. Additionally, dust cap 710 can prevent contamination of the mating sleeve, which could transfer to the connector end face during insertion. When not in use, dust cap 710 can be installed on multi-core fiber optic cable 502 to cover exposed fiber tip 702. Although not depicted, dust cap 710 could be attached to multi-core fiber optic cable 502 via a tether, or other retaining device. In other examples, cortical pin 600 could include a dust cap (also not depicted) or other covering arranged to protect the threads and cavity with which multi-core fiber optic cable 502 is to be mated until such time during use that the multi-core fiber optic cable 502 and cortical pin 600 are to be mated.

FIG. 8 illustrates a surgical navigation system 800, in accordance with non-limiting example(s) of the present disclosure. Surgical navigation system 800 includes a signal generator and receiver 802 and multi-core fiber optic cable 804. Multi-core fiber optic cable 804 can be like the other multi-core fiber optic cables described above (e.g., multi-core fiber optic cable 100, multi-core fiber optic cable 200, multi-core fiber optic cable 400, multi-core fiber optic cable 502, etc.). Multi-core fiber optic cable 804 is coupled to signal generator and receiver 802. In particular, multi-core fiber optic cable 804 is optically coupled to signal generator and receiver 802.

In general, signal generator and receiver 802 is arranged to generate optical signals and transmit the optical signals via the fiber cores of multi-core fiber optic cable 804. Furthermore, signal generator and receiver 802 is arranged to receive optical signals. As described above, multi-core fiber optic cable 804 can include a number of fiber cores and FBGs. The FBGs will reflect a portion of the optical signal transmitted by signal generator and receiver 802. The surgical navigation system 800 can use these reflected optical signals to determine a position and orientation of the patient's bone in real time, or said differently, without conventional imaging. More specifically, a 3D shape of the multi-core fiber optic cable 804 can be determined based on the optical reflections from the FBGs located within the multi-core fiber optic cable 804.

Multi-core fiber optic cable 804 terminates in cable pin assembly 806 and cable pin assembly 808. As depicted in this example, surgical navigation system 800 is arranged to track the position and orientation of a patient's femur 818 and tibia 820 via the cable pin assembly 806 and cable pin assembly 808, respectively, which are fixed to the patient's bones via cortical pins. That is, the shape of the multi-core fiber optic cable, as determined from optical reflections received from the FBGs within the multi-core fiber optic cable 804 can be used to track or determine the position and/or pose of the patient's bones (e.g., the femur 818 and tibia 820, or the like).

Surgical navigation system 800 includes a display 812 and computing system 814. Computing system 814 can include conventional computing elements, such as, processing circuitry and memory. It is noted, that although computing system 814 can be a general-purpose computer, the features and functionality with which computing system 814 are programmed for cannot be accomplished by a human with pen and paper even given significant amounts of time. For example, a human is not capable of reading optical signals from signal generator and receiver 802 and further, a human cannot determine a location of bones of a patient with which cable pin assembly 808 is attached in real time as is needed to use the system intraoperatively.

Computing system 814 is communicatively coupled (e.g., via wired connection, via wireless connection, or the like) to both display 812 and signal generator and receiver 802. During operation, computing system 814 is arranged to receive indications of signals generated by signal generator and receiver 802 and reflected signals received by signal generator and receiver 802 and to generate a representation, or visualization, of the patient's bone, such as, femur 818 and tibia 820. Computing system 814 can cause the generated visualization to be displayed on display 812.

Surgical navigation system 800 can further include a surgical tool 816. Surgical tool 816 can be, for example, a robotic controlled, or robotic assisted surgical tool. As a specific example, surgical tool 816 can be a tool controlled by computing system 814 to provide robotic bone preparation for a surgical procedure (e.g., total knee replacement (TKA), total hip replacement (THA), patellofemoral knee replacement (PFA), unicompartmental knee arthroplasty (UKA), or the like). In conjunction with surgical tool 816, surgical navigation system 800 can include surgical system reference sensor 810 to provide an indication of the position of surgical tool, and to provide a global positional reference for the fiber optic tracking system 816. Said differently, surgical system reference sensor 810 can provide an indication of the position of surgical tool 816 relative to the positions of the cable pin assembly 806 and cable pin assembly 808, and thus, the patient's bones (e.g., femur 818 and tibia 820).

It is noted that with some embodiments, surgical navigation system 800 can be provided without optical tracking elements (e.g., without surgical system reference sensors 810 and associated optical tracking hardware, or the like). In such an example, tracking can be provided solely via multi-core fiber optical cables (e.g., multi-core fiber optic cables 804, or the like). In a specific example, each bony segment (e.g., femur, tibia, etc.) can be tracked by a multi-core fiber optic cable 804 (or a portion, such as an individual fiber of multi-core fiber optic cable 804). In addition, a multi-core fiber optic cable 804, or a portion, such as an individual fiber of multi-core fiber optic cable 804 can be attached to instrumentation (e.g., probe, burr, etc.). It is noted that the reference grating (e.g., FBG sensor 110, or the like) within the fibers can be attached to each tool in a known relative position or known location, thus enabling accurate positioning of the tool and surgical navigation based on the bone and tool locations.

Accordingly, during use, computing system 814 can control (via electronic signaling) surgical tool 816 to cause surgical tool 816 to prepare the patient's bone (e.g., femur 818, tibia 820, or the like) based on the detected position of the femur 818 and tibia 820 relative to the surgical tool 816. With some examples, surgical tool 816 can be controlled (e.g., via computing system 814) to prepare the patient's bone based on a surgery plan provided by a surgeon.

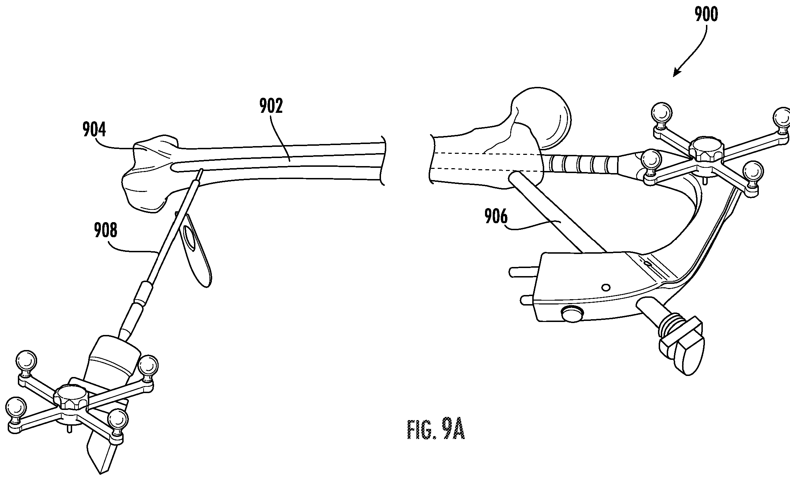

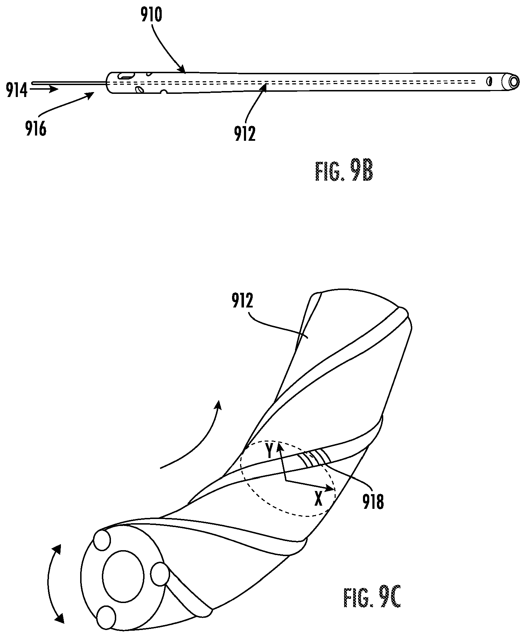

As described above, a surgical navigation system can be provided with improved multi-core fiber optic cables having FBGs arranged to detect a position of a patient's bone without conventional imaging. Also provided herein are systems and methods for utilizing FBGs in other orthopedic applications such as tracking and targeting screw holes in implants for drilling procedures, which are immune to electromagnetic interference. For example, FIG. 9A, FIG. 9B, and FIG. 9C describe detecting implant or instrument deflection within a bone canal via FBGs. More specifically, these figures depict using FBG sensors, such as, described herein, for distal screw hole targeting of an intramedullary (IM) rod, also referred to as an IM nail by accounting for the deflection of the implant in 6DoF. Turning more particularly to FIG. 9A, a system 900 for placing an IM nail 902 in the medullary canal of a bone 904. System 900 further includes fixing device 906 and fixing device 908 for securing the IM nail 902 in the bone 904 via screws (not shown).

A probe including an FBG can be inserted either into the cannulation of the IM nail 902 or a small bore (e.g., a machined recess, or the like) located within the wall of the implant and used to detect deflection of the IM nail 902 in situ. As such, distal hole-targeting for the placement of IM nail 902 can be facilitated with surgical navigation techniques. It also facilitates the ordering of the fixation (distal first, proximal second or vice versa), which is beneficial for certain types of fractures. This removes the need for interoperative radiative imaging to be used to target the screw holes. FIG. 9B depicts a probe 910 with a fiber core 912 that can be inserted into IM nail 902. Fiber core 912 includes one or more FBG 918. During use, a source light signal 914 can be transmitted to the fiber core 912 while return light signal 916 is received from fiber core 912.

As IM nail 902 is inserted into the medullary canal of bone 904, IM nail 902 will deflect. The deflection of IM nail 902 can be measured and/or determined based on return light signal 916 assisting with entry portal acquisition. FIG. 9C depicts a portion of fiber core 912 in a deflected state. As can be seen, as the fiber core 912 moves in either a bending motion or a twisting (e.g., torsional) motion, the amount of bending or twisting can be determined from the return light signal 916. That is, FBG 918 will reflect an amount of source light signal 914 based on the bending or twisting of the fiber core 912. Accordingly, the location of IM nail 902 within bone 904 can be determined. Once IM nail 902 is secured in bone 904, fiber core 912 can be removed.

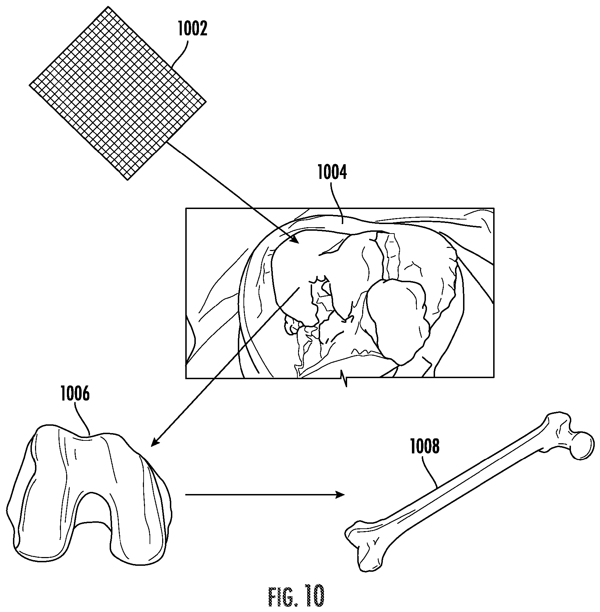

Another embodiment of the present disclosure is an FBG mesh to rapidly characterize/register the anatomy of a patient's bone. FIG. 10 illustrates an example FBG mesh 1002 that can be used to determine the shape of joint surface 1004 of a patient's bone 1008. A shape model 1006 of the joint surface 1004 could be used during navigation assisted surgery, such as, for example, knee resurfacing surgery, or the like.

Intraoperatively, FBG mesh 1002 could be placed on joint surface 1004, such as an articular surface of a bone. Optical signals could be transmitted through fiber cores of the FBG mesh 1002 and return signals received. Based on the return signals, an estimation of the articular shape (e.g., shape of joint surface 1004) could be determined. With some examples, the estimated shape (e.g., shape model 1006, or the like) could be used with intraoperatively captured landmarks to characterize the anatomy of bone 1008. As another example, shape model 1006 could be used as input to a statistical shape model for the particular articular surface to guide the course of treatment.

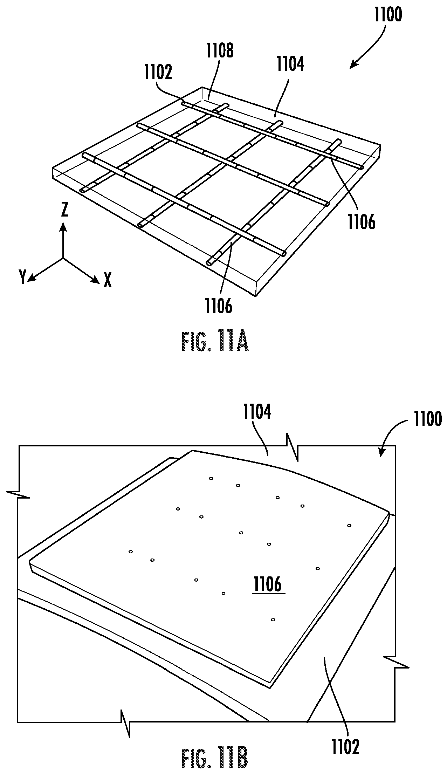

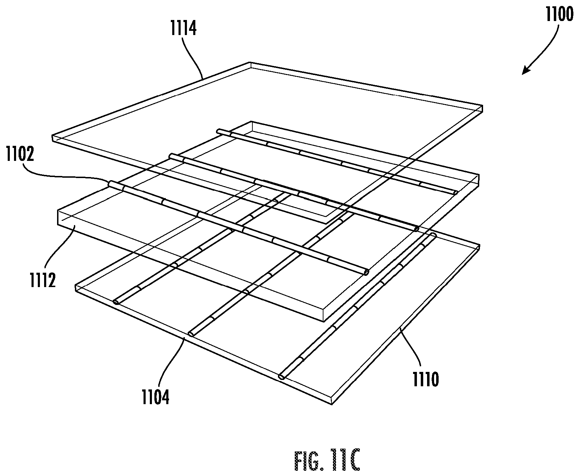

FIG. 11A, FIG. 11B, and FIG. 11C illustrates FBG mesh 1100, in accordance with non-limiting example(s) of the present disclosure. FBG mesh 1100 can be used to characterize a shape, surface, or other anatomy of a patient's intraoperatively, such as, for example, as described with respect to FIG. 10. FBG mesh 1100 includes a number of fiber cores. In particular, FBG mesh 1100 includes fiber cores 1102 and fiber cores 1104 disposed in flexible housing 1108. As depicted in FIG. 11A, fiber cores 1102 are disposed perpendicular to fiber cores 1104, forming a grid. Each of fiber cores 1102 and fiber cores 1104 include one or more FBGs 1106.

Turning to FIG. 11B, fiber cores 1102 and fiber cores 1104 extend out from flexible housing 1108 and are arranged to couple to an optical signal generator and receiver. During use, optical signals can be transmitted through fiber cores 1102 and fiber cores 1104 and return signals can be received. As described previously, the return signals are based on FBGs 1106 and particularly on the state of FBGs 1106. As the FBGs 1106 are disposed in flexible housing 1108 in a grid format, the return optical signals received from each of the fiber cores 1102 and fiber cores 1104 can be used to recreate the shape of a surface with which the FBG mesh 1100, or the flexible housing 1108, is disposed on. Thus, FBG mesh 1100 could be sued with a surgical navigation system (e.g., surgical navigation system 800, or the like) to characterize an anatomy of a patient's bone intraoperatively.

FIG. 11C depicts an alternative embodiment of FBG mesh 1100. As depicted in this figure, FBG mesh 1100 can depicted multiple layers. For example, FBG mesh 1100 can include FBG layer 1110 comprising fiber cores 1104 and associated FBGs 1106. FBG mesh 1100 can further include FBG layer 1112 comprising fiber cores 1102 and associated FBGs 1106. FBG mesh 1100 can additionally, include a protective layer 1114. In some examples, although not pictured here, FBG mesh 1100 can include multiple protective layer 1114, such as, a protective layer 1114 disposed over the upper FBG layer (e.g., FBG layer 1112 and another protective layer 1114 disposed below the lower FBG layer (e.g., FBG layer 1110).

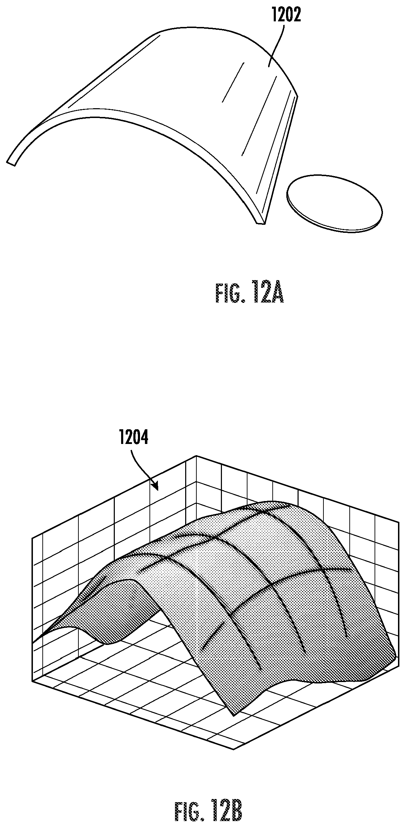

FIG. 12A to FIG. 12F illustrates examples of generating a model or estimation of a shape based on an FBG mesh (e.g., FBG mesh 1002, FBG mesh 1100, or the like), in accordance with non-limiting example(s) of the present disclosure. FIG. 12A depicts a shape 1202 while FIG. 12B depicts a shape model 1204 associated with shape 1202. Shape model 1204 can be generated based on signals from an FBG mesh (e.g., FBG mesh 1002, FBG mesh 1100, or the like) placed over shape 1202. More specifically, optical reflections from FBG sensors within an FBG mesh can be used to determine a specific three-dimensional (3D) geometry of a surface, such as, an articular surface of a patient's bone. In addition to using the fiber-optic mesh-generated to deform a statistical shape model, the same information could be used for intraoperative registration of patient anatomy to preoperative 3D imaging ( CT, MRI, etc.) The fiber-optic generated surface registers the preoperative model to the patient anatomy using one of various surface matching techniques (e.g., ICP, DARCES). This allows the use of preoperative model for surgical navigation and facilitates preoperative planning for navigated procedures.

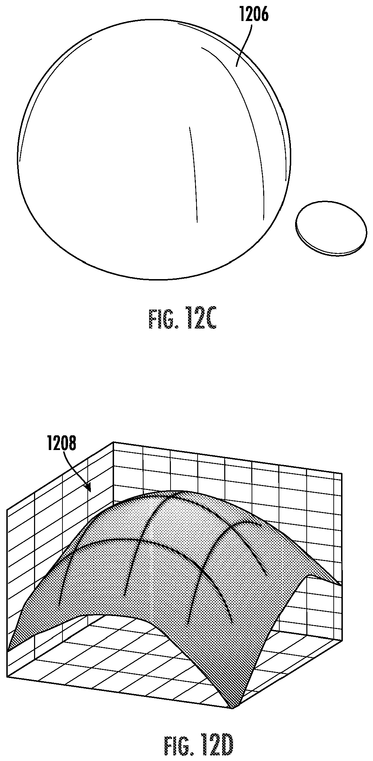

FIG. 12C depicts an example of another shape 1206 while FIG. 12D depicts a shape model 1208 associated with shape 1206. Shape model 1208 can be generated based on signals from an FBG mesh (e.g., FBG mesh 1002, FBG mesh 1100, or the like) placed over shape 1206. More specifically, optical reflections from FBG sensors within an FBG mesh can be used to determine a specific three-dimensional (3D) geometry of a surface, such as, an articular surface of a patient's bone.

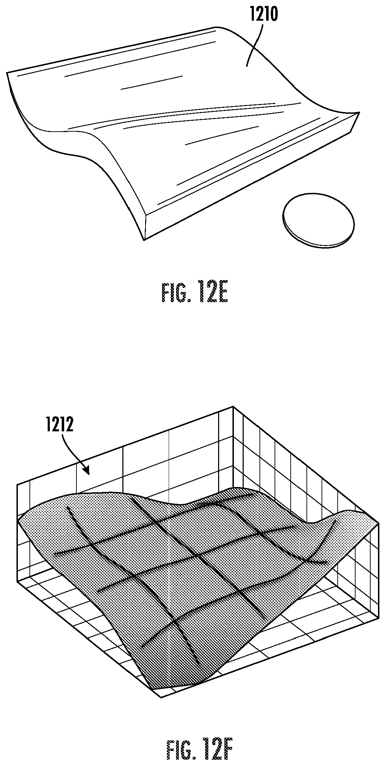

FIG. 12E depicts a shape 1210 while FIG. 12F depicts a shape model 1212 associated with shape 1210. Shape model 1212 can be generated based on signals from an FBG mesh (e.g., FBG mesh 1002, FBG mesh 1100, or the like) placed over shape 1210. More specifically, optical reflections from FBG sensors within an FBG mesh can be used to determine a specific three-dimensional (3D) geometry of a surface, such as, an articular surface of a patient's bone.

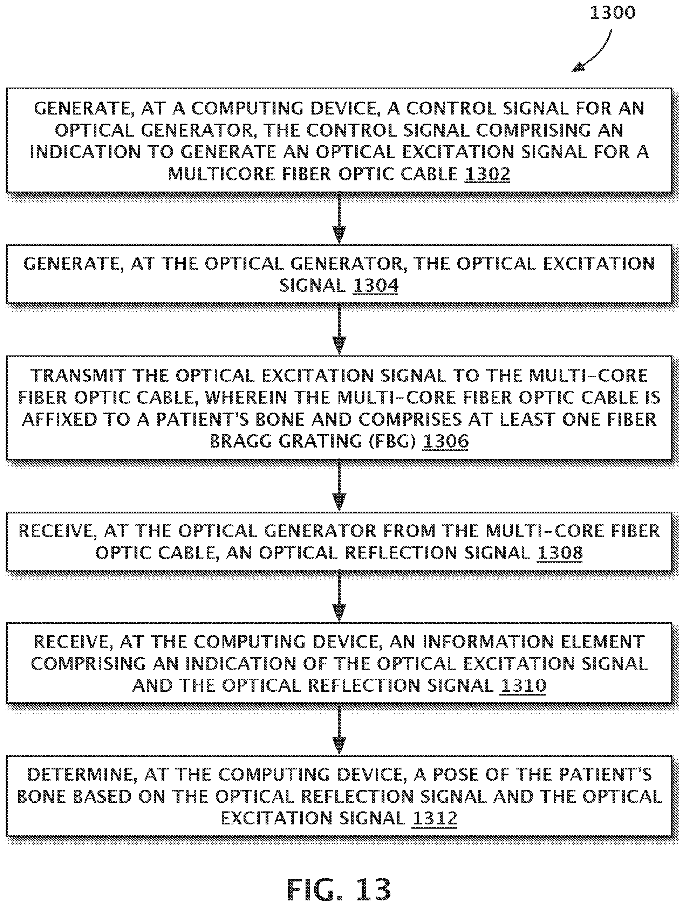

FIG. 13 illustrates a routine 1300, in accordance with non-limiting example(s) of the present disclosure. Routine 1300 can begin at block 1302 "generating, at a computing device, a control signal for an optical generator, the control signal comprising an indication to generate an optical excitation signal for a multi-core fiber optic cable" where a computing device (e.g., computing system 814, machine 1400, or the like) can generate a control signal for an optical generator (e.g., signal generator and receiver 802 or the like). For example, computing system 814 can generate a control signal for signal generator and receiver 802 comprising an indication to generate an optical excitation signal and transmit the optical excitation signal to a multi-core fiber optic cable (e.g., multi-core fiber optic cable 804, or the like). With some examples, signal generator and receiver 802 can be coupled to a multi-core fiber optic cable like multi-core fiber optic cable 804, multi-core fiber optic cable 100, multi-core fiber optic cable 200, multi-core fiber optic cable 400, cable pin assembly 500, or the like. In other examples, signal generator and receiver 802 can be coupled to an FBG mesh such as FBG mesh 1100. With still other examples, signal generator and receiver 802 can be coupled to a multi-core fiber optic cable inserted into an IM nail such as, fiber core 912.

Continuing to block 1304 "generating, at the optical generator, the optical excitation signal" and block 1306 "transmitting the optical excitation signal to the multi-core fiber optic cable, wherein the multi-core fiber optic cable is affixed to a patient's bone and comprises at least one fiber Bragg grates (FBG)" the optical generator can generate the optical excitation signal and transmit the optical excitation signal to a multi-core fiber optic cable. For example, signal generator and receiver 802 can generate an optical signal and transmit the optical signal to multi-core fiber optic cable 804. In some examples, the multi-core fiber optic cable is affixed to a patient's bone, such as, via cable pin assembly 806 and cable pin assembly 808. In other examples, the multi-core fiber optic cable is inserted into an IM nail for insertion into a medullary canal of a patient's bone, such as IM nail 902. In still other examples, the multi-core fiber optic cable can be incorporated into an FBG mesh, such as, FBG mesh 1002, FBG mesh 1100, or the like. In such an example, the FBG mesh can be placed over t patient's bone intraoperatively.

Continuing to block 1308 "receiving, at the optical generator from the multi-core fiber optic cable, an optical reflection signal" the optical generator can receive a return optical signal from the multi-core fiber optic cable. Said differently, the optical generator can receive reflections of the optical excitation signal from the FBG (or FBGs) in the multi-core fiber optic cable. For example, signal generator and receiver 802 can receive optical reflections from FBGs in cable pin assembly 806 and cable pin assembly 808. That is, the optical reflections received by the signal generator can include multiple individual reflection components, such as, for example, a reflection component associated with the FBG in cable pin assembly 806 and another reflection component associated with the FBG in cable pin assembly 808. With other examples, the reflections can be associated with FBGs in an FBG mesh, or an FBG of a fiber core inserted into an IM nail.

Continuing to block 1310 "receiving, at the computing device, an information element comprising an indication of the optical excitation signal and the optical reflection signal" the computing device can receive an information element from the optical generator comprising indications of the optical excitation signal and the optical reflection signal. For example, computing system 814 can receive from signal generator and receiver 802 indications of the optical signals transmitted to multi-core fiber optic cable 804 and the optical signals received from multi-core fiber optic cable 804 (e.g., based on reflections from the FBGs of cable pin assembly 806 and cable pin assembly 808).

Continuing to block 1312 "determining, at the computing device, a pose of the patient's bone based on the optical reflection signal and the optical excitation signal" the computing device can determine a pose of a patient's bone. More particularly, based on the optical excitation signal and the optical reflection signal, the computing device can determine the precise location of the FBGs within multi-core fiber optic cable relative to each other and/or relative to a tracking sensor (e.g., a tracking FBG, a tracking reference sensor, or the like). As another example, the computing device can determine whether the IM nail is properly inserted into the medullary canal of patient's bone. With still another examples, the computing device can determine the shape of a surface of the bone with which the FBG mesh is placed.

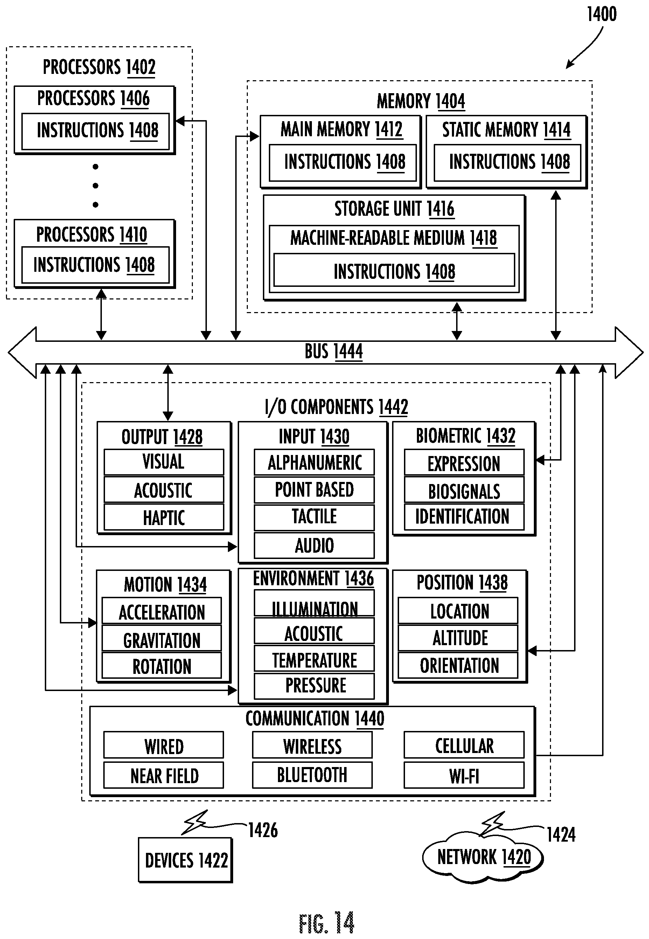

FIG. 14 illustrates a diagrammatic representation of a machine 1400 in the form of a computer system within which a set of instructions may be executed for causing the machine to perform any one or more of the methodologies discussed herein. More specifically, FIG. 14 shows a diagrammatic representation of the machine 1400 in the example form of a computer system, within which instructions 1408 (e.g., software, a program, an application, an applet, an app, or other executable code) for causing the machine 1400 to perform any one or more of the methodologies discussed herein may be executed. For example, the instructions 1408 may cause the machine 1400 to execute routine 1300 of FIG. 13. With some examples, computing system 814 of FIG. 8 can be implemented based on the elements and functionality of machine 1400. More generally, the instructions 1408 may cause the machine 1400 to cooperate with an optical signal generator coupled to a multi-core fiber optic cable to determine a pose of a patient's bone, insertion depth of an IM nail, or a shape of a surface of a patient's bone, as described herein.

The instructions 1408 transform the general, non-programmed machine 1400 into a particular machine 1400 programmed to carry out the described and illustrated functions in a specific manner. In alternative embodiments, the machine 1400 operates as a standalone device or may be coupled (e.g., networked) to other machines. In a networked deployment, the machine 1400 may operate in the capacity of a server machine or a client machine in a server-client network environment, or as a peer machine in a peer-to-peer (or distributed) network environment. The machine 1400 may comprise, but not be limited to, a server computer, a client computer, a personal computer (PC), a tablet computer, a laptop computer, a netbook, a set-top box (STB), a PDA, an entertainment media system, a cellular telephone, a smart phone, a mobile device, a wearable device (e.g., a smart watch), a smart home device (e.g., a smart appliance), other smart devices, a web appliance, a network router, a network switch, a network bridge, or any machine capable of executing the instructions 1408, sequentially or otherwise, that specify actions to be taken by the machine 1400. Further, while only a single machine 1400 is illustrated, the term "machine" shall also be taken to include a collection of machines 200 that individually or jointly execute the instructions 1408 to perform any one or more of the methodologies discussed herein.

The machine 1400 may include processors 1402, memory 1404, and I/O components 1442, which may be configured to communicate with each other such as via a bus 1444. In an example embodiment, the processors 1402 (e.g., a Central Processing Unit (CPU), a Reduced Instruction Set Computing (RISC) processor, a Complex Instruction Set Computing (CISC) processor, a Graphics Processing Unit (GPU), a Digital Signal Processor (DSP), an ASIC, a Radio-Frequency Integrated Circuit (RFIC), another processor, or any suitable combination thereof) may include, for example, a processor 1406 and a processor 1410 that may execute the instructions 1408. The term "processor" is intended to include multi-core processors that may comprise two or more independent processors (sometimes referred to as "cores") that may execute instructions contemporaneously. Although FIG. 14 shows multiple processors 1402, the machine 1400 may include a single processor with a single core, a single processor with multiple cores (e.g., a multi-core processor), multiple processors with a single core, multiple processors with multiples cores, or any combination thereof.

The memory 1404 may include a main memory 1412, a static memory 1414, and a storage unit 1416, both accessible to the processors 1402 such as via the bus 1444. The main memory 1404, the static memory 1414, and storage unit 1416 store the instructions 1408 embodying any one or more of the methodologies or functions described herein. The instructions 1408 may also reside, completely or partially, within the main memory 1412, within the static memory 1414, within machine-readable medium 1418 within the storage unit 1416, within at least one of the processors 1402 (e.g., within the processor's cache memory), or any suitable combination thereof, during execution thereof by the machine 1400.

The I/O components 1442 may include a wide variety of components to receive input, provide output, produce output, transmit information, exchange information, capture measurements, and so on. The specific I/O components 1442 that are included in a particular machine will depend on the type of machine. For example, portable machines such as mobile phones will likely include a touch input device or other such input mechanisms, while a headless server machine will likely not include such a touch input device. It will be appreciated that the I/O components 1442 may include many other components that are not shown in FIG. 14. The I/O components 1442 are grouped according to functionality merely for simplifying the following discussion and the grouping is in no way limiting. In various example embodiments, the I/O components 1442 may include output components 1428 and input components 1430. The output components 1428 may include visual components (e.g., a display such as a plasma display panel (PDP), a light emitting diode (LED) display, a liquid crystal display (LCD), a projector, or a cathode ray tube (CRT)), acoustic components (e.g., speakers), haptic components (e.g., a vibratory motor, resistance mechanisms), other signal generators, and so forth. The input components 1430 may include alphanumeric input components (e.g., a keyboard, a touch screen configured to receive alphanumeric input, a photo-optical keyboard, or other alphanumeric input components), point-based input components (e.g., a mouse, a touchpad, a trackball, a joystick, a motion sensor, or another pointing instrument), tactile input components (e.g., a physical button, a touch screen that provides location and/or force of touches or touch gestures, or other tactile input components), audio input components (e.g., a microphone), and the like.

In further example embodiments, the I/O components 1442 may include biometric components 1432, motion components 1434, environmental components 1436, or position components 1438, among a wide array of other components. For example, the biometric components 1432 may include components to detect expressions (e.g., hand expressions, facial expressions, vocal expressions, body gestures, or eye tracking), measure bio signals (e.g., blood pressure, heart rate, body temperature, perspiration, or brain waves), identify a person (e.g., voice identification, retinal identification, facial identification, fingerprint identification, or electroencephalogram-based identification), and the like. The motion components 1434 may include acceleration sensor components (e.g., accelerometer), gravitation sensor components, rotation sensor components (e.g., gyroscope), and so forth. The environmental components 1436 may include, for example, illumination sensor components (e.g., photometer), temperature sensor components (e.g., one or more thermometers that detect ambient temperature), humidity sensor components, pressure sensor components (e.g., barometer), acoustic sensor components (e.g., one or more microphones that detect background noise), proximity sensor components (e.g., infrared sensors that detect nearby objects), gas sensors (e.g., gas detection sensors to detection concentrations of hazardous gases for safety or to measure pollutants in the atmosphere), or other components that may provide indications, measurements, or signals corresponding to a surrounding physical environment. The position components 1438 may include location sensor components (e.g., a GPS receiver component), altitude sensor components (e.g., altimeters or barometers that detect air pressure from which altitude may be derived), orientation sensor components (e.g., magnetometers), and the like.

Communication may be implemented using a wide variety of technologies. The I/O components 1442 may include communication components 1440 operable to couple the machine 1400 to a network 1420 or devices 1422 via a coupling 1424 and a coupling 1426, respectively. For example, the communication components 1440 may include a network interface component or another suitable device to interface with the network 1420. In further examples, the communication components 1440 may include wired communication components, wireless communication components, cellular communication components, Near Field Communication (NFC) components, Bluetooth^{®} components (e.g., Bluetooth^{®} Low Energy), Wi-Fi^{®} components, and other communication components to provide communication via other modalities. The devices 1422 may be another machine or any of a wide variety of peripheral devices (e.g., a peripheral device coupled via a USB).

Moreover, the communication components 1440 may detect identifiers or include components operable to detect identifiers. For example, the communication components 1440 may include Radio Frequency Identification (RFID) tag reader components, NFC smart tag detection components, optical reader components (e.g., an optical sensor to detect one-dimensional bar codes such as Universal Product Code (UPC) bar code, multi-dimensional bar codes such as Quick Response (QR) code, Aztec code, Data Matrix, Dataglyph, MaxiCode, PDF417, Ultra Code, UCC RSS-2D bar code, and other optical codes), or acoustic detection components (e.g., microphones to identify tagged audio signals). In addition, a variety of information may be derived via the communication components 1440, such as location via Internet Protocol (IP) geolocation, location via Wi-Fi^{®} signal triangulation, location via detecting an NFC beacon signal that may indicate a particular location, and so forth.

The various memories (i.e., memory 1404, main memory 1412, static memory 1414, and/or memory of the processors 1402) and/or storage unit 1416 may store one or more sets of instructions and data structures (e.g., software) embodying or utilized by any one or more of the methodologies or functions described herein. These instructions (e.g., the instructions 1408), when executed by processors 1402, cause various operations to implement the disclosed embodiments.

As used herein, the terms "machine-storage medium," "device-storage medium," "computer-storage medium" mean the same thing and may be used interchangeably in this disclosure. The terms refer to a single or multiple storage devices and/or media (e.g., a centralized or distributed database, and/or associated caches and servers) that store executable instructions and/or data. The terms shall accordingly be taken to include, but not be limited to, solid-state memories, and optical and magnetic media, including memory internal or external to processors. Specific examples of machine-storage media, computer-storage media and/or device-storage media include non-volatile memory, including by way of example semiconductor memory devices, e.g., erasable programmable read-only memory (EPROM), electrically erasable programmable read-only memory (EEPROM), FPGA, and flash memory devices; magnetic disks such as internal hard disks and removable disks; magneto-optical disks; and CD-ROM and DVD-ROM disks. The terms "machine-storage media," "computer-storage media," and "device-storage media" specifically exclude carrier waves, modulated data signals, and other such media, at least some of which are covered under the term "signal medium" discussed below.

In various example embodiments, one or more portions of the network 1420 may be an ad hoc network, an intranet, an extranet, a VPN, a LAN, a WLAN, a WAN, a WWAN, a MAN, the Internet, a portion of the Internet, a portion of the PSTN, a plain old telephone service (POTS) network, a cellular telephone network, a wireless network, a Wi-Fi^{®} network, another type of network, or a combination of two or more such networks. For example, the network 1420 or a portion of the network 1420 may include a wireless or cellular network, and the coupling 1424 may be a Code Division Multiple Access (CDMA) connection, a Global System for Mobile communications (GSM) connection, or another type of cellular or wireless coupling. In this example, the coupling 1424 may implement any of a variety of types of data transfer technology, such as Single Carrier Radio Transmission Technology (1xRTT), Evolution-Data Optimized (EVDO) technology, General Packet Radio Service (GPRS) technology, Enhanced Data rates for GSM Evolution (EDGE) technology, third Generation Partnership Project (3GPP) including 3G, fourth generation wireless (4G) networks, Universal Mobile Telecommunications System (UMTS), High Speed Packet Access (HSPA), Worldwide Interoperability for Microwave Access (WiMAX), Long Term Evolution (LTE) standard, others defined by various standard-setting organizations, other long range protocols, or other data transfer technology.

The instructions 1408 may be transmitted or received over the network 1420 using a transmission medium via a network interface device (e.g., a network interface component included in the communication components 1440) and utilizing any one of a number of well-known transfer protocols (e.g., hypertext transfer protocol (HTTP)). Similarly, the instructions 1408 may be transmitted or received using a transmission medium via the coupling 1426 (e.g., a peer-to-peer coupling) to the devices 1422. The terms "transmission medium" and "signal medium" mean the same thing and may be used interchangeably in this disclosure. The terms "transmission medium" and "signal medium" shall be taken to include any intangible medium that is capable of storing, encoding, or carrying the instructions 1408 for execution by the machine 1400, and includes digital or analog communications signals or other intangible media to facilitate communication of such software. Hence, the terms "transmission medium" and "signal medium" shall be taken to include any form of modulated data signal, carrier wave, and so forth. The term "modulated data signal" means a signal that has one or more of its characteristics set or changed in such a matter as to encode information in the signal.

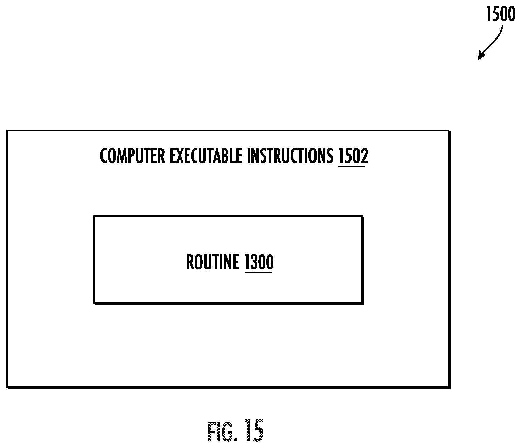

FIG. 15 illustrates computer-readable storage medium 1500. Computer-readable storage medium 1500 may comprise any non-transitory computer-readable storage medium or machine-readable storage medium, such as an optical, magnetic or semiconductor storage medium. In various embodiments, computer-readable storage medium 1500 may comprise an article of manufacture. In some embodiments, 700 may store computer executable instructions 1502 with which circuitry (e.g., a processor of computing system 814, or the liker) can execute. For example, computer executable instructions 1502 can include instructions to implement operations described with respect to routine 1300 of FIG. 13. Examples of computer-readable storage medium 1500 or machine-readable storage medium may include any tangible media capable of storing electronic data, including volatile memory or non-volatile memory, removable or non-removable memory, erasable or non-erasable memory, writeable or re-writeable memory, and so forth. Examples of computer executable instructions 1502 may include any suitable type of code, such as source code, compiled code, interpreted code, executable code, static code, dynamic code, object-oriented code, visual code, and the like.

Terms used herein should be accorded their ordinary meaning in the relevant arts, or the meaning indicated by their use in context, but if an express definition is provided, that meaning controls.

Herein, references to "one embodiment" or "an embodiment" do not necessarily refer to the same embodiment, although they may. Unless the context clearly requires otherwise, throughout the description and the claims, the words "comprise," "comprising," and the like are to be construed in an inclusive sense as opposed to an exclusive or exhaustive sense; that is to say, in the sense of "including, but not limited to." Words using the singular or plural number also include the plural or singular number respectively, unless expressly limited to a single one or multiple ones. Additionally, the words "herein," "above," "below" and words of similar import, when used in this application, refer to this application as a whole and not to any particular portions of this application. When the claims use the word "or" in reference to a list of two or more items, that word covers all of the following interpretations of the word: any of the items in the list, all of the items in the list and any combination of the items in the list, unless expressly limited to one or the other. Any terms not expressly defined herein have their conventional meaning as commonly understood by those having skill in the relevant art(s).

## Claims

1. A surgical navigation system comprising:
a multi-core fiber optic cable (100), comprising:
a plurality of optical fibers (304),
a coupler (116) to optically couple the plurality of optical fibers (304) to an optical generator,
at least one fiber Bragg grating (FBG) (110) in each of the plurality of optical fibers,
at least one tracking sensor portion (108), the at least one tracking sensor portions (108) comprising at least one of the plurality of FBGs (110) and arranged to be affixed to an anatomy of a patient, and
a cable shroud (302) enclosing the plurality of optical fibers (304);
an optical signal generator and receiver (802) optically to be coupled to the plurality of optical fibers (304) in the multi-core fiber optic cable (100), the optical signal generator and receiver (802) to transmit an optical excitation signal to the plurality of optical fibers (304) and to receive a plurality of optical reflection signals from the plurality of optical fibers (304), wherein the plurality of optical reflection signals are generated at least in part by the plurality of FBGs (110); and
a computing system (814) comprising:
processing circuitry; and
memory comprising instructions that when executed by the processing circuitry cause the processing circuitry to:
receive an indication of the optical excitation signal and the plurality of optical reflection signals, and
determine a pose of the anatomy of the patient based in part on the optical excitation signal and the plurality of optical reflection signals,
**characterized in that** the cable shroud (302) comprises a flattened shape, wherein a cross section of the cable shroud (302) comprises a cross-section where a first dimension is longer than a second dimension, the second dimension substantially orthogonal to the first dimension.

2. The surgical navigation system of claim 1, wherein the length of the sensor is less than or equal to 2 meters.

3. The surgical navigation system of any one of claims 1 to 2, wherein the diameter of the sensor is less than or equal to 330 microns.

4. The surgical navigation system of any one of claims 1 to 3, the at least one tracking sensor portion (108) comprising:
an exposed fiber tip (702) arranged to be inserted into a canal (606) of a cortical pin (600); and
a cortical pin coupler (704) arranged to couple to the cortical pin (600) and fix the exposed fiber tip (702) a known distance in the canal (606).

5. The surgical navigation system of any one of claims 1 to 4, the at least one tracking sensor portion (108) comprising a plurality of tracking sensor portions.

6. The surgical navigation system of any one of claims 1 to 5, comprising a surgical system reference sensor (202), the instructions when executed by the processing circuitry cause the processing circuitry to:
identify surgical system coordinates based in part on the surgical system reference sensor (202); and
translate the pose into the surgical system coordinates.

7. The surgical navigation system of clam 6, wherein the surgical system coordinates are three-dimensional cartesian coordinates.

8. The surgical navigation system of any one of claims 1 to 7, the instructions when executed by the processing circuitry cause the processing circuitry to overlay one or more images with the pose of the anatomy of the patient.

9. The surgical navigation system of claim 8, wherein the one or more images may be generated from infrared or computed tomography.

10. The surgical navigation system of any one of claims 1 to 9, the at least one tracking sensor portion (108) comprising a plurality of exposed fiber tips (702) arranged to be inserted into a canal (606) of respective cortical pins (600).

11. The surgical navigation system of any one of claims 1 to 9, wherein the anatomy of the patient is a bone, and wherein the at least one tracking sensor portion (108) is arranged to be coupled to the patient via a bone screws or a bone pin.

12. The surgical navigation system of any one of claims 1 to 9, wherein the at least one tracking sensor portion (108) is arranged to be coupled to the patient via cement, adhesive, or a clamp.

13. The surgical navigation system of claim 1, wherein the multi-core fiber optic cable (100) is inserted into an intramedullary nail.

## Patentansprüche

1. Chirurgisches Navigationssystem, das Folgendes umfasst:
ein Mehrkern-Glasfaserkabel (100), das Folgendes umfasst:
mehrere Glasfasern (304),
einen Koppler (116), um die mehreren Glasfasern (304) mit einem optischen Generator optisch zu koppeln,
mindestens ein Faser-Bragg-Gitter (FBG) (110) in jeder der mehreren Glasfasern,
mindestens einen Verfolgungssensorabschnitt (108), wobei der mindestens eine Verfolgungssensorabschnitt (108) mindestens eines der mehreren FBGs (110) umfasst und dafür ausgelegt ist, an einer Anatomie eines Patienten befestigt zu werden, und
eine Kabelummantelung (302), die die mehreren Glasfasern (304) einschließt;
einen optischen Signalgenerator und -empfänger (802), der an die mehreren Glasfasern (304) in dem Mehrkern-Glasfaserkabel (100) optisch gekoppelt werden soll, wobei der optische Signalgenerator und -empfänger (802) ein optisches Anregungssignal an die mehreren Glasfasern (304) übertragen soll und mehrere optische Reflexionssignale von den mehreren Glasfasern (304) empfangen soll, wobei die mehreren optischen Reflexionssignale zumindest teilweise durch die mehreren FBGs (110) erzeugt werden; und
ein Rechensystem (814), das Folgendes umfasst:
eine Verarbeitungsschaltungsanordnung; und
einen Speicher, der Anweisungen enthält, die dann, wenn sie durch die Verarbeitungsschaltungsanordnung ausgeführt werden, die Schaltungsanordnung veranlassen: ein Anzeichen für das optische Anregungssignal und die mehreren optischen Reflexionssignale zu empfangen, und eine Lage der Anatomie des Patienten teilweise basierend auf dem optischen Anregungssignal und den mehreren optischen Reflexionssignalen zu bestimmen,
**dadurch gekennzeichnet, dass** die Kabelummantelung (302) eine abgeflachte Form umfasst, wobei ein Querschnitt der Kabelummantelung (302) einen Querschnitt umfasst, in dem eine erste Abmessung länger ist als eine zweite Abmessung, wobei die zweite Abmessung im Wesentlichen senkrecht zu der ersten Abmessung ist.

2. Chirurgisches Navigationssystem nach Anspruch 1, wobei die Länge des Sensors kleiner oder gleich 2 Meter ist.

3. Chirurgisches Navigationssystem nach einem der Ansprüche 1 bis 2, wobei der Durchmesser des Sensors kleiner oder gleich 330 Mikrometer ist.

4. Chirurgisches Navigationssystem nach einem der Ansprüche 1 bis 3, wobei der mindestens eine Verfolgungssensorabschnitt (108) Folgendes umfasst:
eine freiliegende Faserspitze (702), die dafür ausgelegt ist, in einen Kanal (606) eines Kortikalstifts (600) eingesetzt zu werden; und
einen Kortikalstiftkoppler (704), der dafür ausgelegt ist, mit dem Kortikalstift (600) zu koppeln und die freiliegende Faserspitze (702) in einem bekannten Abstand in dem Kanal (606) zu fixieren.

5. Chirurgisches Navigationssystem nach einem der Ansprüche 1 bis 4, wobei der mindestens eine Verfolgungssensorabschnitt (108) mehrere Verfolgungssensorabschnitte umfasst.

6. Chirurgisches Navigationssystem nach einem der Ansprüche 1 bis 5, das einen Referenzsensor (202) für das chirurgische System umfasst, wobei die Anweisungen dann, wenn sie durch die Schaltungsanordnung ausgeführt werden, die Schaltungsanordnung dazu veranlassen:
Koordinaten des chirurgischen Systems teilweise basierend auf dem Referenzsensor (202) für das chirurgische System zu identifizieren; und
die Lage in Koordinaten des chirurgischen Systems zu übersetzen.

7. Chirurgisches Navigationssystem nach Anspruch 6, wobei die Koordinaten des chirurgischen Systems dreidimensionale kartesische Koordinaten sind.

8. Chirurgisches Navigationssystem nach einem der Ansprüche 1 bis 7, wobei die Anweisungen dann, wenn sie durch die Schaltungsanordnung ausgeführt werden, die Schaltungsanordnung dazu veranlassen, ein oder mehrere Bilder mit der Lage der Anatomie des Patienten zu überlagern.

9. Chirurgisches Navigationssystem nach Anspruch 8, wobei das eine oder die mehreren Bilder aus Infrarot- oder Computertomographie erzeugt werden können.

10. Chirurgisches Navigationssystem nach einem der Ansprüche 1 bis 9, wobei der mindestens eine Verfolgungssensorabschnitt (108) mehrere freiliegende Faserspitzen (702) umfasst, die dafür ausgelegt sind, in einen Kanal (606) der entsprechenden Kortikalstifte (600) eingesetzt zu werden.

11. Chirurgisches Navigationssystem nach einem der Ansprüche 1 bis 9, wobei die Anatomie des Patienten ein Knochen ist und wobei der mindestens eine Verfolgungssensorabschnitt (108) dafür ausgelegt, über Knochenschrauben oder einen Knochenstift an den Patienten gekoppelt zu werden.

12. Chirurgisches Navigationssystem nach einem der Ansprüche 1 bis 9, wobei der mindestens eine Verfolgungssensorabschnitt (108) dafür ausgelegt ist, über Zement, Klebemittel oder eine Klemmvorrichtung an den Patienten gekoppelt zu werden.

13. Chirurgisches Navigationssystem nach Anspruch 1, wobei das Mehrkern-Glasfaserkabel (100) in einen intramedullären Nagel eingesetzt wird.

## Revendications

1. Système de navigation chirurgicale, comprenant :
un câble à fibres optiques multicœur (100), comprenant :
une pluralité de fibres optiques (304),
un coupleur (116) pour coupler optiquement la pluralité de fibres optiques (304) à un générateur optique,
au moins un réseau de Bragg (FBG) sur fibre (110) dans chacune de la pluralité de fibres optiques,
au moins une partie capteur de suivi (108), l'au moins une partie capteur de suivi (108) comprenant au moins un de la pluralité de FBG (110) et étant agencée de manière à être fixée à l'anatomie d'un patient, et
une enveloppe de câble (302) enfermant la pluralité de fibres optiques (304) ;
un générateur et un récepteur de signal optique (802) destinés à être couplés optiquement à la pluralité de fibres optiques (304) dans le câble à fibres optiques multicœur (100), le générateur et le récepteur de signal optique (802) étant destinés à transmettre un signal d'excitation optique à la pluralité de fibres optiques (304) et à recevoir une pluralité de signaux de réflexion optique provenant de la pluralité de fibres optiques (304), la pluralité de signaux de réflexion optique étant générés au moins en partie par la pluralité de FBG (110) ; et
un système informatique (814) comprenant :
des circuits de traitement ; et
une mémoire comprenant des instructions qui, lorsqu'elles sont exécutées par les circuits de traitement, amènent les circuits de traitement à :
recevoir une indication du signal d'excitation optique et de la pluralité de signaux de réflexion optique, et déterminer une pose de l'anatomie du patient en partie sur la base du signal d'excitation optique et de la pluralité de signaux de réflexion optique,
**caractérisé en ce que** l'enveloppe de câble (302) comprend une forme aplatie, une section transversale de l'enveloppe de câble (302) comprenant une section transversale où une première dimension est plus longue qu'une deuxième dimension, la deuxième dimension étant sensiblement orthogonale à la première dimension.

2. Système de navigation chirurgical selon la revendication 1, la longueur du capteur étant inférieure ou égale à 2 mètres.

3. Système de navigation chirurgical selon l'une quelconque des revendications 1 à 2, le diamètre du capteur étant inférieur ou égal à 330 microns.

4. Système de navigation chirurgical selon l'une quelconque des revendications 1 à 3, l'au moins une partie de capteur de suivi (108) comprenant :
une pointe de fibre exposée (702) agencée pour être insérée dans un canal (606) d'une broche corticale (600) ; et
un coupleur à broche corticale (704) agencé pour se coupler à la broche corticale (600) et fixer la pointe de fibre exposée (702) à une distance connue dans le canal (606).

5. Système de navigation chirurgical selon l'une quelconque des revendications 1 à 4, l'au moins une partie de capteur de suivi (108) comprenant une pluralité de parties de capteur de suivi.

6. Système de navigation chirurgical selon l'une quelconque des revendications 1 à 5, comprenant un capteur de référence de système chirurgical (202), les instructions, lorsqu'elles sont exécutées par les circuits de traitement, amenant les circuits de traitement à :
identifier les coordonnées du système chirurgical en partie sur la base du capteur de référence du système chirurgical (202) ; et
traduire la pose en coordonnées du système chirurgical.

7. Système de navigation chirurgical selon la revendication 6, les coordonnées du système chirurgical étant des coordonnées cartésiennes tridimensionnelles.

8. Système de navigation chirurgicale selon l'une quelconque des revendications 1 à 7, les instructions, lorsqu'elles sont exécutées par le circuit de traitement, amenant le circuit de traitement à recouvrir une ou plusieurs images avec la pose de l'anatomie du patient.

9. Système de navigation chirurgicale selon la revendication 8, la ou les images pouvant être générées à partir de tomographie infrarouge ou par ordinateur.

10. Système de navigation chirurgical selon l'une quelconque des revendications 1 à 9, l'au moins une partie de capteur de suivi (108) comprenant une pluralité de pointes de fibres exposées (702) agencées pour être insérées dans un canal (606) de broches corticales respectives (600).

11. Système de navigation chirurgicale selon l'une quelconque des revendications 1 à 9, l'anatomie du patient étant un os, et l'au moins une partie de capteur de suivi (108) étant agencée pour être couplée au patient par l'intermédiaire d'une vis à os ou d'une broche à os.

12. Système de navigation chirurgical selon l'une quelconque des revendications 1 à 9, l'au moins une partie de capteur de suivi (108) étant agencée pour être couplée au patient par l'intermédiaire d'un ciment, d'un adhésif ou d'une pince.

13. Système de navigation chirurgical selon la revendication 1, le câble à fibres optiques multicœur (100) étant inséré dans un clou intramédullaire.
